# EUROPEAN PATENT APPLICATION

(11) **EP 2 009 434 A2**
(43) Date of publication of application: **31.12.2008**
(21) Application number: 08158131.6
(22) Date of filing: 12.06.2008
(51) Int. Cl.: G01N 27/22, G01N 33/28

(54) **System and Methods for Determining a Concentration of Biodiesel in a mixture of Biodiesel and Petrodiesel**

(30) Priority: 29.06.2007 US 771516
(71) Applicant: Delphi Technologies, Inc., Troy, Michigan 48007 (US)
(72) Inventor: Lin, Yingjie, El Paso, TX 79912 (US); Wang, Su-Chee S., Troy, MI 48098 (US); Wu, Ming-Cheng, Troy, MI 48090 (US)
(74) Representative: Denton, Michael John

(57) **Abstract**

Systems and methods for determining a concentration of biodiesel in a mixture of biodiesel and petrodiesel are provided. In one exemplary embodiment, a method includes receiving an oscillatory signal at an inductance-capacitance-resistance circuit (20). The circuit (20) has a sensing element (42) fluidly communicating with the mixture of biodiesel and petrodiesel. The method further includes generating a resonant current at a resonant frequency utilizing the circuit (20) in response to the oscillatory signal. The method further includes determining a concentration value indicating the concentration of the biodiesel in the mixture based on an amplitude of the resonant current and/or the resonant frequency, utilizing a microprocessor (30). The method further includes storing the concentration value in a memory device (32), utilizing the microprocessor (30).

## Description

### BACKGROUND OF THE INVENTION

Fourier transform infrared (FT-IR) spectrometers have been utilized to determine a concentration of biodiesel in a blend of diesel fuel. However, substantial drawbacks with laboratory-use FT-IR spectrometers include a relatively high cost (e.g., greater than $80,000) and an inability to accurately measure a concentration of biodiesel when moisture impurities are in the blend of diesel fuel. Further, accurate analysis of a biodiesel concentration is often performed in a controlled laboratory environment.

Accordingly, the inventors herein have recognized a need for a system and a method for determining a concentration of biodiesel in a mixture of biodiesel and petrodiesel that minimizes and/or eliminates the above mentioned deficiencies.

### SUMMARY OF THE INVENTION

A method for determining a concentration of biodiesel in a mixture of biodiesel and petrodiesel in accordance with the exemplary embodiment is provided. The method includes receiving an oscillatory signal at an inductance-capacitance-resistance circuit. The circuit has a sensing element fluidly communicating with the mixture of biodiesel and petrodiesel. The method further includes generating a resonant current at a resonant frequency utilizing the circuit in response to the oscillatory signal. The method further includes determining a concentration value indicating the concentration of the biodiesel in the mixture based on an amplitude of the resonant current, utilizing a microprocessor. The method further includes storing the concentration value in a memory device, utilizing the microprocessor.

A system for determining a concentration of biodiesel in a mixture of biodiesel and petrodiesel in accordance with another exemplary embodiment is provided. The system includes an inductance-capacitance-resistance circuit configured to receive an oscillatory signal. The circuit has a sensing element fluidly communicating with the mixture of biodiesel and petrodiesel. The circuit is further configured to generate a resonant current at a resonant frequency in response to the oscillatory signal. The system further includes a microprocessor operatively associated with the circuit. The microprocessor is configured to determine a concentration value indicating the concentration of the biodiesel in the mixture based on an amplitude of the resonant current. The microprocessor is further configured to store the concentration value in a memory device.

A method for determining a concentration of biodiesel in a mixture of biodiesel and petrodiesel in accordance with another exemplary embodiment is provided. The method includes receiving an oscillatory signal at an inductance-capacitance-resistance circuit. The circuit has a sensing element fluidly communicating with the mixture of biodiesel and petrodiesel. The method further includes generating a resonant current at a resonant frequency utilizing the circuit in response to the oscillatory signal. The method further includes determining a dielectric constant value indicating a dielectric constant associated with the biodiesel in the mixture based on the resonant frequency of the resonant current, utilizing a microprocessor. The method further includes determining a concentration value indicating the concentration of the biodiesel in the mixture based on the dielectric constant value, utilizing the microprocessor. The method further includes storing the concentration value in a memory device, utilizing the microprocessor.

A system for determining a concentration of biodiesel in a mixture of biodiesel and petrodiesel in accordance with another exemplary embodiment is provided. The system includes an inductance-capacitance-resistance circuit configured to receive an oscillatory signal. The circuit has a sensing element fluidly communicating with the mixture of biodiesel and petrodiesel. The circuit is further configured to generate a resonant current at a resonant frequency in response to the oscillatory signal. The system further includes a microprocessor operatively associated with the circuit. The microprocessor is configured to determine a dielectric constant value indicating a dielectric constant associated with the biodiesel in the mixture based on the resonant frequency of the resonant current. The microprocessor is further configured to determine a concentration value indicating the concentration of the biodiesel in the mixture based on the dielectric constant value. The microprocessor is further configured to store the concentration value in a memory device.

A method for determining a concentration of biodiesel in a mixture of biodiesel and petrodiesel in accordance with another exemplary embodiment is provided. The method includes receiving an oscillatory signal at an inductance-capacitance-resistance circuit. The circuit has a sensing element fluidly communicating with the mixture of biodiesel and petrodiesel. The method further includes generating a resonant current at a resonant frequency utilizing the circuit in response to the oscillatory signal. The method further includes determining a dielectric constant value indicating a dielectric constant associated with the biodiesel in the mixture based on the resonant frequency of the resonant current, utilizing a microprocessor. The method further includes determining an index value based on the dielectric constant value and an amplitude of the resonant current, utilizing the microprocessor. The method further includes determining a concentration value indicating a concentration of the biodiesel in the mixture based on the index value, utilizing the microprocessor. The method further includes storing the concentration value in a memory device, utilizing the microprocessor.

A system for determining a concentration of biodiesel in a mixture of biodiesel and petrodiesel in accordance with another exemplary embodiment. The system includes an inductance-capacitance-resistance circuit configured to receive an oscillatory signal. The circuit has a sensing element fluidly communicating with the mixture of biodiesel and petrodiesel. The circuit is further configured to generate a resonant current at a resonant frequency in response to the oscillatory signal. The system further includes a microprocessor operatively associated with the circuit. The microprocessor is configured to determine a dielectric constant value indicating a dielectric constant associated with the biodiesel in the mixture based on the resonant frequency of the resonant current. The microprocessor is further configured to determine an index value based on the dielectric constant value and an amplitude of the resonant current. The microprocessor is further configured to determine a concentration value indicating a concentration of the biodiesel in the mixture based on the index value. The microprocessor is further configured to store the concentration value in a memory device.

### BRIEF DESCRIPTION OF THE DRAWINGS

Figure 1 is a schematic of a system for determining a concentration of biodiesel in a mixture of biodiesel and petrodiesel in accordance with an exemplary embodiment;
Figure 2 is a schematic of a sensing element utilized in the system of Figure 1;
Figure 3 is an electrical schematic corresponding to the sensing element of Figure 2;
Figure 4 is a graph having curves illustrating a relationship between a biodiesel concentration and an amplitude of a resonant current in the sensing element of Figure 2 wherein the graph has an x-axis corresponding to a biodiesel concentration and a y-axis corresponding to an amplitude of a resonant current;
Figure 5 is a graph having curves illustrating a relationship between biodiesel concentration and a dielectric constant associated with the sensing element of Figure 2 wherein the graph has an x-axis corresponding to a biodiesel concentration and a y-axis corresponding to a dielectric constant associated with the sensing element;
Figure 6 is a graph having curves illustrating a delta resonant current and a delta dielectric constant associated the sensing element of Figure 2 for biodiesel oxidized for a predetermined time wherein the graph has an x-axis corresponding to a biodiesel concentration and a y-axis corresponding to both the delta resonant current and the delta dielectric constant associated with the sensing element of Figure 2;
Figure 7 is a graph having curves wherein the graph has an x-axis corresponding to a biodiesel concentration and a y-axis corresponding to (delta resonant current)² / delta dielectric constant;
Figure 8 is a graph having curves wherein the graph has an x-axis corresponding to a biodiesel concentration and a y-axis corresponding to (delta resonant current)² / delta dielectric constant;
Figure 9 is a table indicating a concentration of fatty acids in biodiesel obtained from a soy feedstock, a cottonseed feedstock, and a poultry fat feedstock;
Figure 10 is a flowchart of a method for determining a concentration of biodiesel in a mixture of biodiesel and petrodiesel in accordance with another exemplary embodiment;
Figure 11 is a flowchart of a method for determining a concentration of biodiesel in a mixture of biodiesel and petrodiesel in accordance with another exemplary embodiment; and
Figure 12 is a flowchart of a method for determining a concentration of biodiesel in a mixture of biodiesel and petrodiesel in accordance with another exemplary embodiment.

### DETAILED DESCRIPTION OF EXEMPLARY EMBODIMENTS

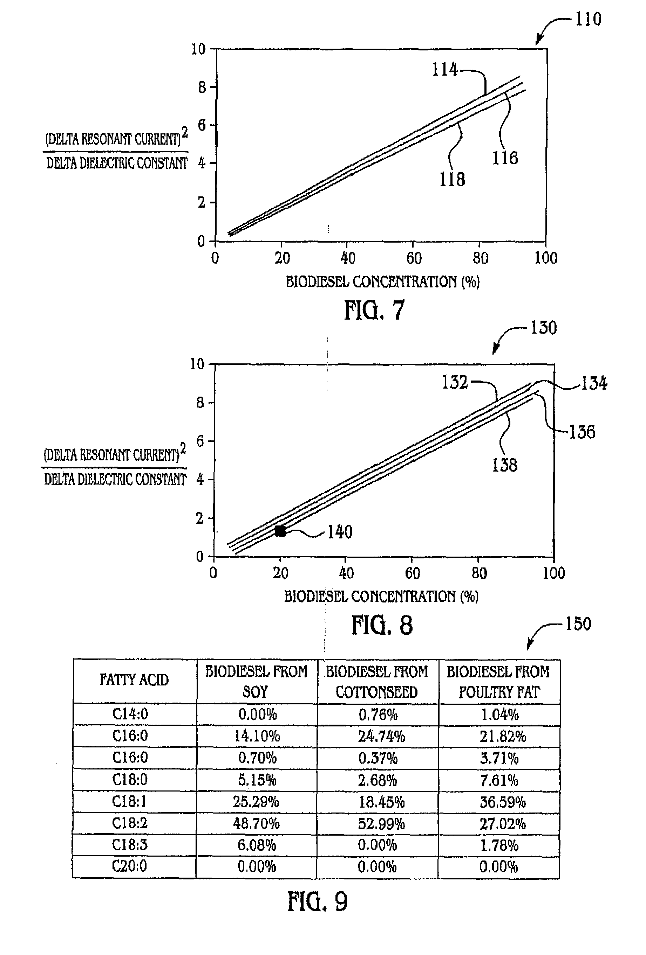

Biodiesel can be obtained from several different source feedstocks. For example, biodiesel can be obtained from soy feedstock, cottonseed feedstock, and poultry fat feedstock. Further, referring to Figure 9, biodiesel obtained from differing source feedstocks include several fatty acids in differing concentrations. For example, the table 150 indicates that: (i) biodiesel obtained from soy feedstock has a 0% concentration by volume of the fatty acid C14:0, (ii) biodiesel obtained from a cottonseed feedstock has a 0.76% concentration by volume of the fatty acid C14:0, and (iii) biodiesel obtained from a poultry fat feedstock has a 1.04% concentration by volume of the fatty acid C14:0.

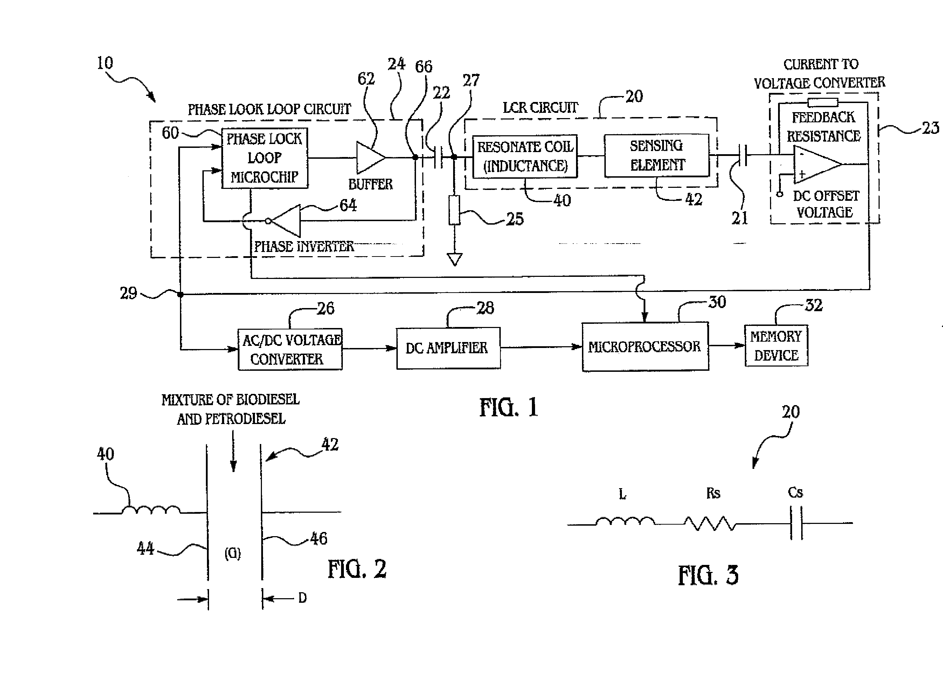

Referring to Figure 1, a system 10 for determining a concentration of biodiesel in a mixture of biodiesel and petrodiesel in accordance with an exemplary embodiment is illustrated. The system 10 includes an inductance-capacitance-resistance (LCR) circuit 20, capacitors 21, 22, a current-to-voltage converter 23, a phase lock loop circuit 24, a resistor 25, an alternating current (AC)/direct current (DC) voltage converter 26, a DC amplifier 28, a microprocessor 30, and a memory device 32. An advantage of the system 10 is that the system can accurately determine a concentration of biodiesel in a mixture irrespective of the source feedstock used to obtain the biodiesel and irrespective of water and insoluble components being in the mixture. It should be noted that the term "L" corresponds to inductance, the term "R" corresponds to resistance, and the term "C" corresponds to capacitance herein. It should be further noted that the term "index value" corresponds to any value which is indicative of a concentration of biodiesel.

Referring to Figures 1-3, the LCR circuit 20 is provided to generate an oscillatory signal having an amplitude indicative of a concentration of biodiesel in a mixture of biodiesel and petrodiesel. The LCR circuit 20 includes an inductor 40 electrically coupled in series with a sensing element 42. The sensing element 42 includes electrodes 44 and 46. The electrodes 44, 46 are spaced apart from one another a distance (D) and are constructed of an electrically conductive material. For example, the electrodes 44, 46 can be constructed from one or more of steel, copper, brass, gold, silver, titanium, or alloys thereof. A portion of a mixture of biodiesel and petrodiesel is received in a region (G) disposed between the electrodes 44, 46. When an oscillatory signal at a predetermined frequency is received by the LCR circuit 20, the LCR circuit 20 outputs a resonant current at a resonant frequency. In one exemplary embodiment, the oscillatory signal received by the LCR circuit 20 is in a frequency range of 1-10 Mhz. The amplitude of the resonant current output by the LCR circuit 20 at the electrode 46 has an amplitude indicative of the concentration of biodiesel in the mixture of biodiesel and petrodiesel disposed between the electrodes 44, 46. The resonant current from the LCR circuit 20 is received by the current-to-voltage converter 23 via the capacitor 21. As shown, the LCR circuit 20 is electrically coupled in series between a node 27 and the capacitor 21. Referring to Figure 3, the LCR circuit 20 can be represented by an equivalent circuit having an inductance (L), a resistance (Rs), and a capacitance (Cs) electrically coupled in series with one another.

Referring to Figure 1, the current-to-voltage converter 23 is provided to generate an output voltage signal in response to the resonant current from the LCR circuit 20, which is received by both the phase lock loop circuit 24 and the AC/DC voltage converter 26. The current-to-voltage converter 23 is electrically coupled in series between the capacitor 21 and a node 29.

The phase lock loop circuit 24 is provided to generate an oscillatory signal that is received by the LCR circuit 20. The phase lock loop circuit 24 includes a phase lock loop microchip 60, a buffer 62, and a phase inverter 64. The phase lock loop circuit 24 receives signals from both the current-to-voltage converter 23 and a phase inverter 64. The phase lock loop circuit 24 outputs the oscillatory signal that is transmitted through the buffer 62 to a node 66. From the node 66, the oscillatory signal propagates through the capacitor 22 to the LCR circuit 20 to stimulate the LCR circuit 20. Further, from the node 66 the oscillatory signal propagates to the phase inverter 64. The phase inverter 64 modifies a phase of the oscillatory signal which is received by the phase lock loop microchip 60. During operation, the phase lock loop microchip 60 generates the oscillatory signal that is received by the LCR circuit 20 to induce the LCR circuit 20 to output a resonant current at a resonant frequency. Further, the phase lock loop microchip 60 sends a message to the microprocessor 30 having data indicating a resonant frequency of the output oscillatory signal from the phase lock loop circuit 24, that is further indicative of a resonant frequency of the resonant current of the LCR circuit 20.

It should be noted that in an alternative embodiment of the system 10, the phase lock loop circuit 24 could be replaced with a signal generator (not shown) that receives a signal from the current-to-voltage converter 23 and adjusts an output oscillatory signal is received by the LCR circuit 20 based on the signal from the current-to-voltage converter 23.

A node 27 is electrically coupled to both the capacitor 22 and the LCR circuit 20. A resistor 25 is electrically coupled between the node 27 and electrical ground. The resistor 25 is provided to reset a mean value of the oscillatory signal from the phase lock loop circuit 24 to a ground level.

The AC/DC voltage converter 26 is provided to receive the voltage signal from the current-to-voltage converter 23 and to generate a DC voltage signal indicative of an amplitude of the received voltage signal. The AC/DC voltage converter 26 is electrically coupled between the node 29 and the DC amplifier 28.

The DC amplifier 28 is provided to amplify the DC voltage signal received from the AC/DC voltage converter 26 and to send the amplified DC voltage signal to the microprocessor 30. The DC amplifier 28 is electrically coupled between the AC/DC voltage converter 26 and the microprocessor 30.

The microprocessor 30 is provided to determine a concentration value indicative of a concentration of biodiesel in a mixture of biodiesel and petrodiesel. In particular, the microprocessor 30 is configured to execute software algorithms to determine the concentration of biodiesel in the mixture based on (i) a dielectric constant associated with the mixture, (ii) an amplitude of the resonant current of the LCR circuit 20, or (iii) both the dielectric constant associated with the mixture and the amplitude of the resonant current of the LCR circuit 20, as will be explained in greater detail below. The microprocessor is further configured to store the concentration value in the memory device 32. As shown, the microprocessor 30 is electrically coupled to the DC amplifier 28, the memory device 32, and the phase lock loop microchip 60.

Before providing a detailed explanation of the methodology for determining a concentration of biodiesel in a mixture of biodiesel and petrodiesel, an explanation of the physical properties that can be utilized to determine the biodiesel concentration will be discussed.

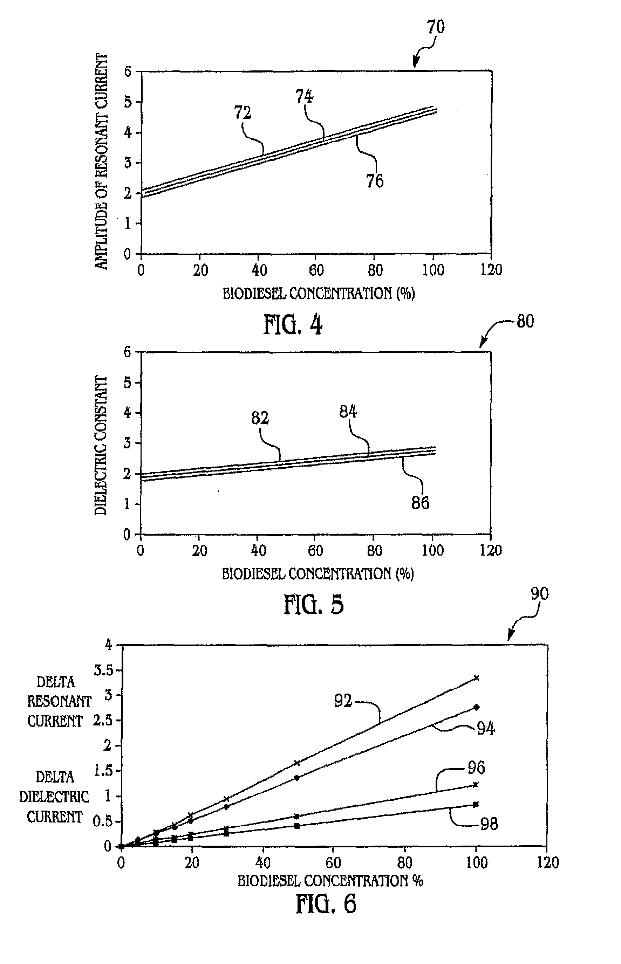

Referring to Figure 4, a graph 70 having curves 72, 74, 76 is illustrated. The graph 70 has an x-axis corresponding to a biodiesel concentration and a y-axis corresponding to an amplitude of a resonant current from the LCR circuit 20 is illustrated. The curve 72 corresponds to fresh biodiesel obtained from a soy feedstock. The curve 74 (almost identical to curve 72) corresponds to fresh biodiesel obtained from a poultry fat feedstock. The curve 76 (almost identical to curves 72 and 74) corresponds to fresh biodiesel obtained from a cottonseed feedstock. The curves 72, 74, 76 illustrate a substantially linear relationship between the biodiesel concentration and an amplitude of the resonant current. Thus, by measuring an amplitude of the resonant current of the LCR circuit 20, the concentration of biodiesel in the mixture of biodiesel and petrodiesel can be determined utilizing data corresponding to one or more of the curves 72, 74, 76. It should be noted that one or more of the curves 72, 74, 76 can be utilized to determine a concentration of biodiesel where the biodiesel is obtained from a soy feedstock, a poultry fat feedstock, or a cottonseed feedstock, for example.

Referring to Figure 5, a graph 80 having curves 82, 84, 86 is illustrated. The graph 80 has an x-axis corresponding to a biodiesel concentration and a y-axis corresponding to a dielectric constant associated with the sensing element 42 and a mixture of biodiesel and petrodiesel is illustrated. The curve 82 corresponds to fresh biodiesel obtained from a soy feedstock. The curve 84 (almost identical to curve 82) corresponds to fresh biodiesel obtained from a poultry fat feedstock. The curve 86 (almost identical to curves 82 and 84) corresponds to fresh biodiesel obtained from a cottonseed feedstock. As shown, the curves 82, 84, 86 illustrate a substantially linear relationship between the biodiesel concentration and the dielectric constant. Thus, by measuring the dielectric constant associated with the sensing element 42 and the mixture of biodiesel and petrodiesel, the concentration ofbiodiesel can be determined utilizing data corresponding to one or more of the curves 82, 84, 86. It should be noted that one or more of the curves 82, 84, 86 can be utilized to determine a concentration of biodiesel where the biodiesel is obtained from a soy feedstock, a poultry fat feedstock, or a cottonseed feedstock, for example.

Referring to Figure 6, a graph 90 having curves 92, 94, 96 and 98 is illustrated. The curve 92 represents a delta resonant current associated with a mixture of fresh (e.g., non-oxidized) biodiesel and petrodiesel versus a biodiesel concentration. The delta resonant current is obtained by subtracting a resonant current amplitude associated with pure petrodiesel from a resonant current amplitude associated with a mixture of biodiesel and petrodiesel. The curve 94 represents a delta resonant current associated with a mixture of baked (e.g., oxidized) biodiesel and petrodiesel versus a biodiesel concentration. As shown by curves 92, 94, the relationship between the delta resonant current and the biodiesel concentration can vary based upon whether the biodiesel is oxidized or non-oxidized.

The curve 96 represents a delta dielectric constant associated with a mixture of fresh (e.g., non-oxidized) biodiesel and petrodiesel versus a biodiesel concentration. The delta dielectric constant is obtained by subtracting a dielectric constant associated with pure petrodiesel from a dielectric constant associated with a mixture of biodiesel and petrodiesel. The curve 98 represents a delta dielectric constant associated with a mixture of baked (e.g., oxidized) biodiesel and petrodiesel versus a biodiesel concentration. As shown by curves 96, 98, the relationship between the delta dielectric constant and the biodiesel concentration can vary based upon whether the biodiesel is oxidized or non-oxidized.

Referring to Figure 7, a graph 110 having curves 114, 116, 118 is illustrated. The graph 110 has an x-axis corresponding to a biodiesel concentration and a y-axis corresponding to (delta resonant current)² / delta dielectric constant. The curve 114 corresponds to fresh biodiesel obtained from a soy feedstock. The curve 116 corresponds to fresh biodiesel obtained from a poultry fat feedstock. The curve 118 corresponds to fresh biodiesel obtained from a cottonseed feedstock. As shown, the linear characteristics including the slope of the curves 114, 116, 118 are a substantially similar to one another. Thus, by determining a value corresponding to (delta resonant current)² / delta dielectric constant, the concentration of biodiesel in a mixture of biodiesel and petrodiesel can be determined utilizing data corresponding to one or more of the curves 114, 116, 118.

Referring to Figure 8, a graph 130 having curves 132, 134, 136, 138, 140 is illustrated. The graph 130 has an x-axis corresponding to a biodiesel concentration and a y-axis corresponding to (delta resonant current)² / delta dielectric constant. The curve 138 corresponds to fresh biodiesel obtained from a soy feedstock. The curve 134 corresponds to biodiesel obtained from a soy feedstock that has been oxidized for 12 hours. The curve 136 corresponds to biodiesel obtained from a soy feedstock that has been oxidized five hours. The curve 132 corresponds to biodiesel with 1500 parts-per-million water by volume in biodiesel obtained from a soy feedstock. The single data point 140 (shown as a square marker at around 20% biodiesel concentration) corresponds to biodiesel with insoluble components therein. Thus, by determining a value corresponding to (delta resonant current)² / delta dielectric constant, the concentration of biodiesel in a mixture of biodiesel and petrodiesel can be determined utilizing data corresponding to one or more of the curves 132, 134, 136, 138, irrespective of water or insoluble components being in the mixture.

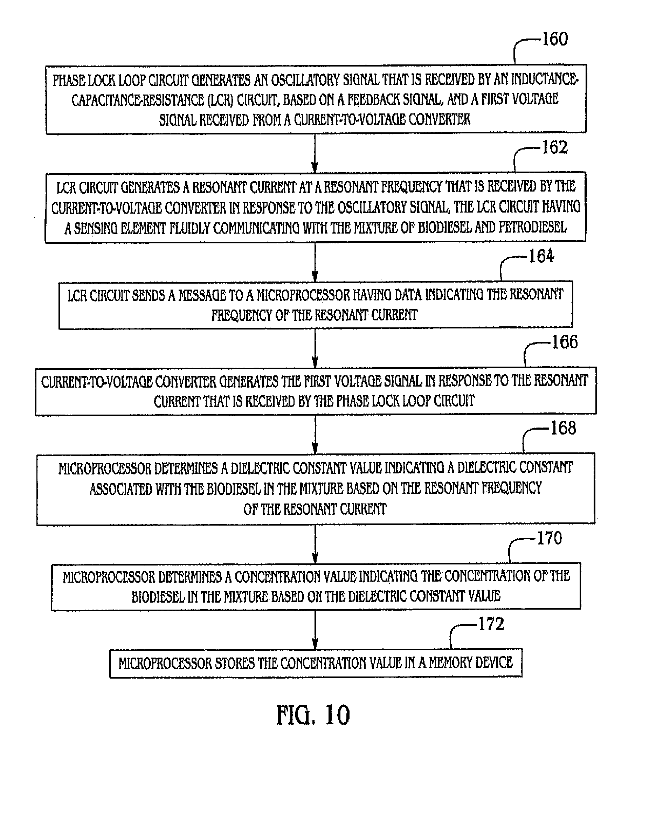

Referring to Figure 10, a flowchart of a method for determining a concentration of biodiesel and a mixture of biodiesel and petrodiesel based on an amplitude of a resonant current in accordance with another exemplary embodiment will now be explained. The method can be implemented in part utilizing software algorithms executed by the microprocessor 30.

At step 160, the phase lock loop circuit 24 generates an oscillatory signal that is received by the LCR circuit 20, based on a feedback signal, and a first voltage signal from the current-to-voltage converter 23.

At step 162, the LCR circuit 20 generates a resonant current at a resonant frequency that is received by the current-to-voltage converter 23 in response to the oscillatory signal. The LCR circuit 20 has the sensing element 42 fluidly communicating with a mixture of biodiesel and petrodiesel.

At step 164, the current-to-voltage converter 23 generates the first voltage signal in response to the resonant current that is received by both the phase lock loop circuit 24 and the AC/DC voltage converter 26.

At step 166, the AC/DC voltage converter 26 generates a second voltage signal in response to the first voltage signal.

At step 168, the DC amplifier 28 amplifies the second voltage signal to obtain a third voltage signal that is received by the microprocessor 30. The third signal is indicative of an amplitude of the resonant current.

At step 170, the microprocessor 30 determines a concentration value indicating the concentration of the biodiesel in the mixture based on the third voltage signal. For example, the microprocessor 30 can utilize a lookup table stored in the memory device 32 having data corresponding to the graph 70 and the curves 72, 74, 76 to determine the biodiesel concentration based upon the third voltage signal indicating the amplitude of the resonant current.

At step 172, the microprocessor 30 stores the concentration value in the memory device 32. After step 172, the method is exited.

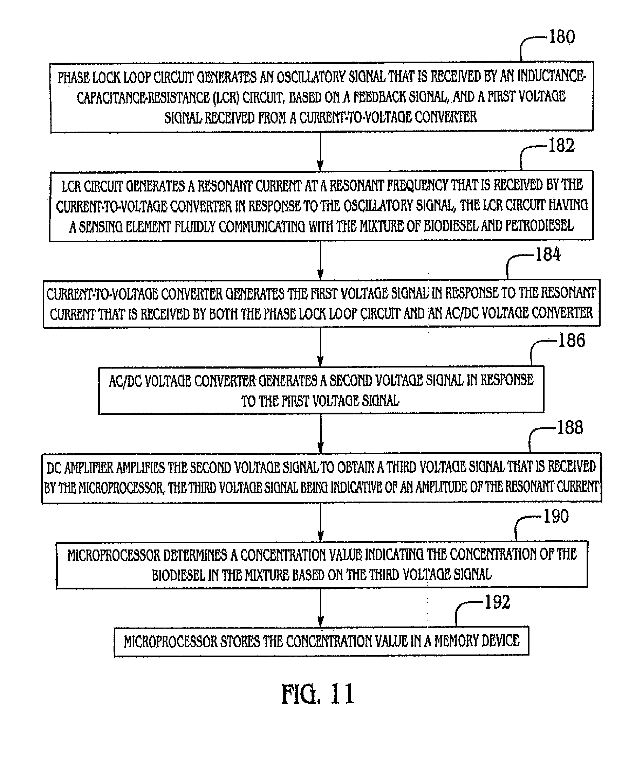

Referring to Figure 11, a flowchart of a method for determining a concentration of biodiesel in a mixture ofbiodiesel and petrodiesel based on a dielectric constant in accordance with another exemplary embodiment will now be explained. The method can be implemented in part utilizing software algorithms executed by the microprocessor 30.

At step 180, the phase lock loop circuit 24 generates an oscillatory signal that is received by the LCR circuit 20, based on a feedback signal, and a first voltage signal from the current-to-voltage converter 23.

At step 182, the LCR circuit 20 generates a resonant current at a resonant frequency that is received by the current-to-voltage converter 23 in response to the oscillatory signal. The LCR circuit 20 has the sensing element 42 fluidly communicating with a mixture of biodiesel and petrodiesel.

At step 184, the LCR circuit 20 sends a message to the microprocessor 30 having data indicating the resonant frequency of the resonant current.

At step 186, the current-to-voltage converter 23 generates the first voltage signal in response to the resonant current that is received by the phase lock loop circuit 24.

At step 188, the microprocessor 30 determines a dielectric constant value indicating a dielectric constant associated with the biodiesel in the mixture based on the resonant frequency of the resonant current. It should be noted that the relationship between the resonant frequency of the resonant current and the dielectric constant can be empirically determined by one skilled in the art.

At step 190, the microprocessor 30 determines a concentration value indicating the concentration of the biodiesel in the mixture based on the dielectric constant value. For example, the microprocessor 30 can utilize a lookup table stored in the memory device 32 having data corresponding to the graph 80 and the curves 82, 84, 86 to determine the biodiesel concentration based on the dielectric constant value.

At step 192, the microprocessor 30 stores the concentration value in the memory device 32.

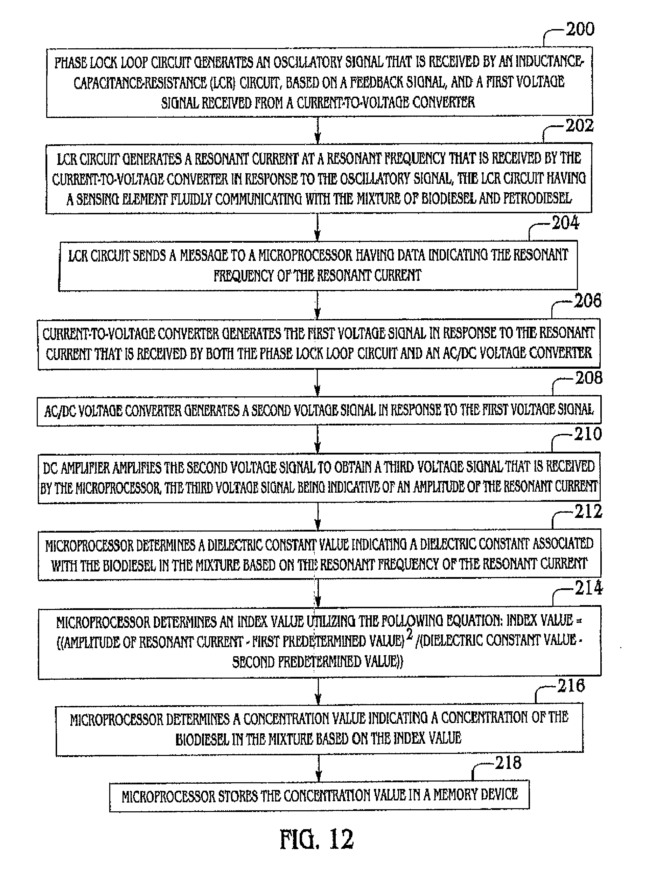

Referring to Figure 12, a flowchart of a method for determining a concentration of biodiesel in a mixture ofbiodiesel and petrodiesel based on a resonant current and a dielectric constant in accordance with another exemplary embodiment will now be explained. The method can be implemented in part utilizing software algorithms executed by the microprocessor 30.

At step 200, the phase lock loop circuit 24 generates an oscillatory signal that is received by the LCR circuit, based on a feedback signal, and a first voltage signal from the current-to-voltage converter 23.

At step 202, the LCR circuit 20 generates a resonant current at a resonant frequency that is received by the current-to-voltage converter 23 in response to the oscillatory signal. The LCR circuit 20 has the sensing element 42 fluidly communicating with a mixture of biodiesel and petrodiesel.

At step 204, the LCR circuit 20 sends a message to the microprocessor 30 having data indicating the resonant frequency of the resonant current.

At step 206, the current-to-voltage converter 23 generates the first voltage signal in response to the resonant current that is received by both the phase lock loop circuit 24 and an AC/DC voltage converter 26.

At step 208, the AC/DC voltage converter 26 generates a second voltage signal in response to the first voltage signal.

At step 210, the DC amplifier 28 amplifies the second voltage signal to obtain a third voltage signal that is received by the microprocessor 30. The third signal is indicative of an amplitude of the resonant current.

At step 212, the microprocessor 30 determines a dielectric constant value indicating a dielectric constant associated with the biodiesel in the mixture based on the resonant frequency of the resonant current.

At step 214, the microprocessor 30 determines an index value utilizing the following equation: index value = ((amplitude of resonant current - first predetermined value)² / (dielectric constant value - second predetermined value)). The first predetermined value corresponds to amplitude of a resonant current when pure petrodiesel is being measured. The second predetermined value corresponds to a dielectric constant value when pure petrodiesel is being measured. It should be noted that the value (amplitude of resonant current - first predetermined value) corresponds to the delta resonant current discussed above. It should be further noted that the value (dielectric constant value - second predetermined value) corresponds to the delta dielectric constant discussed above.

At step 216, the microprocessor 30 determines a concentration value indicating a concentration of the biodiesel in the mixture based on the index value. For example, the microprocessor 30 can utilize a lookup table stored in the memory device 32 and access a concentration value in the lookup table based upon the index value.

At step 218, the microprocessor 30 stores the concentration value in the memory device 32. After step 218, the method is exited.

The systems and methods for determining a concentration of biodiesel in a mixture of biodiesel and petrodiesel represent a substantial advantage over other systems and methods. In particular, the systems and methods provide a technical effect of accurately measuring the concentration of biodiesel in a mixture of biodiesel and petrodiesel utilizing a relatively small inexpensive sensing element. Further, the system and methods are able to accurately measure a concentration of biodiesel in the mixture of biodiesel and petrodiesel irrespective of moisture impurities and insoluble components being in the mixture.

While the invention has been described with reference to exemplary embodiments, it will be understood by those skilled in the art that various changes may be made and equivalents may be substituted for elements thereof without departing from the scope of the invention. In addition, many modifications may be made to adapt a particular situation or material to the teachings of the invention without departing from the essential scope thereof. Therefore, it is intended that the invention not be limited to the particular embodiments disclosed for carrying this invention, but that the invention will include all embodiments falling within the scope of the appended claims. Moreover, the use of the terms, first, second, etc. are used to distinguish one element from another. Furthermore, the use of the terms a, an, etc. do not denote a limitation of quantity, but rather denote the presence of at least one of the referenced items.

## Claims

1. A method for determining a concentration of biodiesel in a mixture of biodiesel and petrodiesel, comprising:
receiving an oscillatory signal at an inductance-capacitance-resistance circuit (20), the circuit (20) having a sensing element (42) fluidly communicating with the mixture of biodiesel and petrodiesel;
generating a resonant current at a resonant frequency utilizing the circuit (20) in response to the oscillatory signal;
determining a concentration value indicating the concentration of the biodiesel in the mixture based on an amplitude of the resonant current, utilizing a microprocessor (30); and
storing the concentration value in a memory device (32), utilizing the microprocessor (30).

2. The method of claim 1, wherein the oscillatory signal has a frequency in a range of 1-10 Mhz.

3. A system (10) for determining a concentration of biodiesel in a mixture ofbiodiesel and petrodiesel, comprising:
an inductance-capacitance-resistance circuit (20) configured to receive an oscillatory signal, the circuit having a sensing element (42) fluidly communicating with the mixture of biodiesel and petrodiesel, the circuit further configured to generate a resonant current at a resonant frequency in response to the oscillatory signal;
a microprocessor (30) operatively associated with the circuit, the microprocessor (30) configured to determine a concentration value indicating the concentration of the biodiesel in the mixture based on an amplitude of the resonant current, the microprocessor (30) further configured to store the concentration value in a memory device (32).

4. The system of claim 3, wherein the oscillatory signal has a frequency in a range of 1-10 Mhz.

5. A method for determining a concentration of biodiesel in a mixture of biodiesel and petrodiesel, comprising:
receiving an oscillatory signal at an inductance-capacitance-resistance circuit (20), the circuit (20) having a sensing element (42) fluidly communicating with the mixture of biodiesel and petrodiesel;
generating a resonant current at a resonant frequency utilizing the circuit (20) in response to the oscillatory signal;
determining a dielectric constant value indicating a dielectric constant associated with the biodiesel in the mixture based on the resonant frequency of the resonant current, utilizing a microprocessor (30);
determining a concentration value indicating the concentration of the biodiesel in the mixture based on the dielectric constant value, utilizing the microprocessor (30); and
storing the concentration value in a memory device (32), utilizing the microprocessor (30).

6. The method of claim 5, wherein the oscillatory signal has a frequency in a range of 1-10 Mhz.

7. A system for determining a concentration of biodiesel in a mixture of biodiesel and petrodiesel, comprising:
an inductance-capacitance-resistance circuit (20) configured to receive an oscillatory signal, the circuit (20) having a sensing element (42) fluidly communicating with the mixture of biodiesel and petrodiesel, the circuit (20) further configured to generate a resonant current at a resonant frequency in response to the oscillatory signal; and
a microprocessor (30) operatively associated with the circuit (20), the microprocessor (30) configured to determine a dielectric constant value indicating a dielectric constant associated with the biodiesel in the mixture based on the resonant frequency of the resonant current, the microprocessor (30) further configured to determine a concentration value indicating the concentration of the biodiesel in the mixture based on the dielectric constant value, and the microprocessor (30) further configured to store the concentration value in a memory device (32).

8. The system of claim 7, wherein the oscillatory signal has a frequency in a range of 1-10 Mhz.

9. A method for determining a concentration of biodiesel in a mixture of biodiesel and petrodiesel, comprising:
receiving an oscillatory signal at an inductance-capacitance-resistance circuit (20), the circuit (20) having a sensing element (42) fluidly communicating with the mixture of biodiesel and petrodiesel;
generating a resonant current at a resonant frequency utilizing the circuit (20) in response to the oscillatory signal;
determining a dielectric constant value indicating a dielectric constant associated with the biodiesel in the mixture based on the resonant frequency of the resonant current, utilizing a microprocessor (30);
determining an index value based on the dielectric constant value and an amplitude of the resonant current, utilizing the microprocessor (30);
determining a concentration value indicating a concentration of the biodiesel in the mixture based on the index value, utilizing the microprocessor (30); and
storing the concentration value in a memory device (32), utilizing the microprocessor (30).

10. The method of claim 9, wherein the oscillatory signal has a frequency in a range of 1-10 Mhz.

11. A system (10) for determining a concentration of biodiesel in a mixture ofbiodiesel and petrodiesel, comprising:
an inductance-capacitance-resistance circuit (20) configured to receive an oscillatory signal, the circuit having a sensing element (42) fluidly communicating with the mixture of biodiesel and petrodiesel, the circuit (20) further configured to generate a resonant current at a resonant frequency in response to the oscillatory signal; and
a microprocessor (30) operatively associated with the circuit (20), the microprocessor (30) configured to determine a dielectric constant value indicating a dielectric constant associated with the biodiesel in the mixture based on the resonant frequency of the resonant current, the microprocessor (30) further configured to determine an index value based on the dielectric constant value and an amplitude of the resonant current, the microprocessor (30) further configured to determine a concentration value indicating a concentration of the biodiesel in the mixture based on the index value, the microprocessor (30) further configured to store the concentration value in a memory device (32).

12. The system of claim 11, wherein the oscillatory signal has a frequency in a range of 1-10 Mhz.
